(19) Europäisches Patentamt
European Patent Office
Office européen des brevets

(11) Numéro de publication : **0 086 726**
**B1**

# (12) FASCICULE DE BREVET EUROPÉEN

(45) Date de publication du fascicule du brevet : **26.06.85**

(51) Int. Cl.[4] : **C 07 C 85/24**, C 07 C 87/60

(21) Numéro de dépôt : **83420010.7**

(22) Date de dépôt : **20.01.83**

## (54) Procédé de préparation de métahalogéno anilines.

(30) Priorité : **05.02.82 FR 8202021**

(43) Date de publication de la demande : **24.08.83 Bulletin 83/34**

(45) Mention de la délivrance du brevet : **26.06.85 Bulletin 85/26**

(84) Etats contractants désignés : **AT BE CH DE FR GB IT LI LU NL SE**

(56) Documents cités :
**EP-A- 0 015 219**
**FR-A- 2 298 531**
**GB-A- 2 042 542**

(73) Titulaire : **RHONE-POULENC AGROCHIMIE**
**14-20, rue Pierre Baizet**
**F-69009 Lyon (FR)**

(72) Inventeur : **Cordier, Georges**
**Chemin des Hermières**
**F-69340 Francheville (FR)**

(74) Mandataire : **Chrétien, François et al**
**RHONE-POULENC AGROCHIMIE BP 9163**
**F-69263 Lyon Cedex 09 (FR)**

**EP 0 086 726 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention se rapporte à un procédé de préparation d'anilines substituées par un halogène en position méta par action de l'hydrogène sur des composés aromatiques aminés plus fortement halogénés. Ces anilines métahalogéno sont des intermédiaires notamment pour la fabrication de produits phytosanitaires.

La préparation de chloranilines substituées en position méta par réaction de polychloranilines en milieu acide avec de l'hydrogène sous pression, en présence d'un catalyseur à base de métal noble, a été décrite dans le brevet français 2 298 531. Le procédé décrit nécessite toutefois l'utilisation de pressions élevées et de quantités très importantes d'acide chlorhydrique, ce qui pose de graves problèmes de corrosion.

La présente invention a précisément pour but de préparer des halogénoanilines substituées en méta par hydrodéchloration sélective de polyhalogénoanilines, sans problème de corrosion.

L'invention concerne plus particulièrement un procédé de préparation d'anilines substituées en position méta par un atome d'halogène par hydrogénation catalytique en phase liquide, organique anhydre en présence d'un acide à chaud et sous pression, en présence de métaux nobles du groupe VIII de la classification périodique, de dérivés benzéniques aminés et chlorés, de formule :

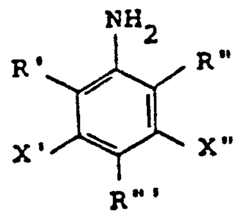

(I)

dans laquelle

X′ et X″, identiques ou différents entre eux, représentent chacun un atome d'halogène, de préférence de chlore, ou un radical alcoyle de 1 à 4 atomes de carbone ou alcoxyle de 1 à 4 atomes de carbone, au moins l'un des X′ et X″ étant obligatoirement un atome d'halogène, de préférence de chlore, l'un des X′ et X″ pouvant, de plus, être l'hydrogène,

R′, R″ et R‴, identiques ou différents entre eux, représentent chacun un atome d'halogène, de préférence de chlore, ou un radical alcoyle de 1 à 4 atomes de carbone ou alcoxyle de 1 à 4 atomes de carbone, benzyle ou phényle éventuellement halogéné, ou phénoxyle, l'un au moins de ces trois symboles représentant l'atome d'halogène et de préférence de chlore, au plus deux des R′, R″ ou R‴ pouvant, de plus, être l'hydrogène,

caractérisé en ce que la réaction est effectuée en présence d'un hydracide, notamment halogéné, dans un solvant chlorobenzénique.

Par hydracide halogéné on entend un hydracide anhydre contenant un atome d'halogène tel que chlore et de préférence brome et iode.

Cet acide peut être ajouté tel quel dès le début de la réaction ou sous forme d'un générateur qui, dans les conditions de réaction, libère progressivement l'acide. Comme générateur on utilise de préférence l'halogène correspondant à l'acide ou un halogénure d'un métal alcalin ou d'ammonium quaternaire.

Cet acide ou son générateur sous forme liquide, essentiellement anhydre ou gazeux, soluble dans le milieu, est ajouté en une quantité, qui dépend de la nature de l'acide, telle que le rapport molaire par rapport à la polychloraniline de départ est généralement compris entre 1/100 et 10/1 et de préférence entre 1/10 et 5/1.

Le procédé selon l'invention s'effectue en phase liquide (hormis bien sûr le catalyseur à base de métal noble) organique anhydre homogène constitué par une solution, dans les conditions de la réaction, de la polychloroaniline de départ dans un solvant, ou mélange de solvants, inerte, du type chlorobenzène. Tous les chlorobenzènes liquides dans les conditions de réaction, c'est-à-dire mono, di, tri, tétra et penta, conviennent mais pour plus de commodité on donne la préférence à ceux qui sont liquides à la température ambiante à savoir le monochlorobenzène, les dichloro-1,2 et 1,3 benzène et le tri-chloro-1,2,4 benzène.

La pression, à laquelle s'effectue la réaction, est généralement supérieure à 5 bars (pression relative). Il n'y a pas de limite supérieure critique pour la pression mais pour des raisons d'ordre économique, il est avantageux d'opérer à des pressions inférieures à 100 bars, les pressions inférieures à 20 bars étant préférées.

La température de réaction est généralement comprise entre 90 et 300 °C, de préférence entre 160 et 230 °C. Les températures élevées, quoique peu économiques, ne sont pas impossibles, étant donné le caractère très peu corrosif du milieu réactionnel anhydre et que les solvants utilisés n'induisent que des pressions faibles à ces températures.

Les métaux nobles constituant la base des catalyseurs utilisés dans l'invention sont des métaux du groupe VIII de la classification périodique tels que le ruthénium, le rhodium, le palladium, l'osmium,

l'iridium et le platine ; le palladium est le métal préféré. Le métal peut être à l'état métallique ou à l'état de composé chimique ; généralement on préfère que le métal soit mis en œuvre à l'état métallique.

Le catalyseur peut être supporté ou non supporté. Comme support du catalyseur, on peut utiliser tout support connu en soi pour supporter des catalyseurs, pourvu que ce support soit résistant au milieu et aux acides ; comme support convenant plus particulièrement, on peut citer le charbon actif, l'alumine, la silice, le sulfate de baryum ; le charbon actif est un support préféré. Le catalyseur ainsi que son support sont avantageusement sous forme finement divisée ; des surfaces spécifiques supérieures à 100 $m^2/g$ conviennent généralement bien.

La quantité de catalyseur mise en œuvre est telle que la proportion pondérale de métal noble du catalyseur par rapport au composé de formule (I) à traiter est généralement comprise entre 0,01 et 15 %, de préférence entre 0,5 et 10 %.

Par ailleurs, le métal noble peut être associé à un autre métal codéposé avec lui sur le support. Ce second métal appartient aux groupes 1b à 5a de la classification périodique. On peut citer en particulier le bismuth, le plomb, l'étain, le thallium, le mercure et l'argent. La demanderesse a notamment constaté que de bons résultats sont obtenus en utilisant l'argent.

Comme composés de formule (I) susceptibles d'être traités par le procédé de l'invention, on peut citer de préférence : la dichloro-2,3 aniline, la dichloro-2,5 aniline, la dichloro-3,4 aniline, la trichloro-2,3,4 aniline, la trichloro-2,3,5 aniline, la trichloro-2,3,6 aniline, la trichloro-2,4,5 aniline, la trichloro-3,4,5 aniline, la tétrachloro-2,3,4,6 aniline, la tétrachloro-2,3,4,5 aniline, la tétrachloro-2,3,5,6 aniline, la pentachloroaniline ; mais aussi : la trichloro-4,5,6 méthyl-2 aniline, la dichloro-2,5 méthyl-4 aniline, la tétrachloro-2,3,5,6 méthyl-4 aniline, la dichloro-2,5 éthyl-4 aniline ; la dichloro-2,5 propyl-4 aniline, la trichloro-3,4,6 benzyl-2 aniline, le diamino-2,2′ hexachloro-3,5,6,3′,5′,6′ diphénylméthane, l'amino-2 trichloro-3,4,5 diphényle, le diamino-4,4′ octachlorodiphényle, la dichloro-4,5 méthoxy-2 aniline, la dichloro-3,4 méthoxy-2 aniline, la dichloro-3,6 méthoxy-2 aniline, la dichloro-5,6 méthoxy-2 aniline, la trichloro-5,6 méthoxy-2 aniline, la trichloro-3,4,6 méthoxy-2 aniline, la trichloro-3,4,5 méthoxy-2 aniline, la tétrachloro-3,4,5 méthoxy-2 aniline, la tétrachloro-3,4,5,6 méthoxy-2 aniline, la dichloro-4,5 méthoxy-3 aniline, la dichloro-2,5 méthoxy-3 aniline, la trichloro-4,5,6 méthoxy-3 aniline, la tétrachloro-2,4,5,6 méthoxy-3 aniline, la dichloro-2,3 méthoxy-4 aniline, la dichloro-2,5 méthoxy-4 aniline, la trichloro-2,3,6 méthoxy-4 aniline, la trichloro-2,3,5 méthoxy-4 aniline, la tétrachloro-2,3,5,6 méthoxy-4 aniline, la dichloro-4,5 phénoxy-2 aniline, la tétrachloro-3,4,5,6 phénoxy-2 aniline, la dichloro-2,5 phénoxy-4 aniline, la tétrachloro-2,3,5,6 phénoxy-4 aniline.

Parmi les anilines substituées en position méta par l'atome de chlore et susceptibles d'être préparées par le procédé selon l'invention, on peut citer de préférence : la métachloraniline ; la dichloro-3,5 aniline mais aussi la chloro-5 méthyl-2 aniline ; la chloro-5 méthyl-3 aniline ; la chloro-3 méthyl-4 aniline ; la dichloro-3,5 méthyl-4 aniline ; la chloro-5 diméthyl-3,4 aniline ; la chloro-3 éthyl-4 aniline ; la chloro-3 benzyl-2 aniline ; le diamino-4,4′ tétrachloro-2,6,2′,6′ diphényle ; la chloro-3 méthoxy-2 aniline ; la chloro-5 méthoxy-2 aniline ; la dichloro-3,5 méthoxy-2 aniline ; la chloro-3 méthoxy-4 aniline ; la chloro-4 méthoxy-3 aniline ; la dichloro-3,5 méthoxy-4 aniline ; la chloro-3 phénoxy-2 aniline ; la chloro-5 phénoxy-2 aniline ; la dichloro-3,5 phénoxy-2 aniline ; la dichloro-3,5 phénoxy-4 aniline.

Le procédé selon l'invention peut être effectué en continu ou en discontinu. En fin de réaction, le catalyseur peut être séparé, le cas échéant, par filtration ou par des moyens équivalents tels que l'essorage ; l'aniline métachlorée préparée peut être séparée par tout moyen connu en soi, par exemple par extraction à l'aide d'un solvant et/ou par distillation.

Le procédé selon l'invention est très avantageux car il permet d'obtenir des anilines métachlorées dans d'excellentes conditions de sélectivité, à des températures et sous des pressions modérées, et ce sans souci de corrosion importante et d'usure prématurée du matériel.

Les exemples suivants, donnés à titre non limitatif, illustrent la mise en œuvre du procédé selon l'invention et les résultats obtenus.

## Exemple 1

Dans un autoclave de 125 $cm^3$ en acier inoxydable, on charge :

$4 \times 10^{-3}$ mole de trichloro-3,4,5-aniline,

20 ml de trichloro-1,2,4-benzène,

0,2 g d'un catalyseur constitué de palladium déposé sur du charbon actif (surface spécifique 1 300 $m^2/g$, teneur pondérale en palladium 5 %).

On élimine l'oxygène de l'autoclave par des purges à l'azote puis à l'hydrogène. On introduit ensuite à l'aide d'une réserve contenant de l'acide chlorhydrique anhydre sous pression, $4 \times 10^{-3}$ mole d'HCl puis on porte la pression de l'autoclave à 9 bars (à température ambiante) avec de l'hydrogène. On chauffe à 210 °C et laisse réagir pendant 6 heures à cette température. La pression s'élève à 15 bars environ. L'autoclave est alors refroidi, dégazé et vidé. Le mélange réactionnel est traité par de l'eau additionnée de soude en quantité telle que tout l'acide chlorhydrique présent (dissous ou combiné aux polychloroanilines) soit neutralisé.

On filtre le catalyseur, on le lave à l'eau et au trichlorobenzène.

La phase organique est analysée par chromatographie en phase vapeur.

Dans ces conditions on observe que le taux de transformation de la trichloro-3,4,5 aniline est de 100 % et le rendement en dichloro-3,5 aniline de 96,5 %. Il s'est aussi formé 3,5 % de chloro-3 aniline. Le taux de déchloration du solvant est de 0,16 % en mole d'HCl par raport au solvant.

### Exemple 2

On répète l'exemple 1 sous 20 bars d'hydrogène (mesurés à l'ambiante) soit environ 35 bars à 210 °C. Pour un taux de transformation de 100 % de la trichloro-3,4,5 aniline, le rendement en dichloro-3,5 aniline est de 97,9 % et celui de la chloro-3 aniline de 2,1 %. Le taux d'hydrodéchloration du solvant est de 0,16 %.

### Exemple 3

On répète l'exemple 1 sauf qu'on charge $40 \times 10^{-3}$ moles d'HCl anhydre et 23 bars d'$H_2$ mesurés à l'ambiante (la pression à 210 °C est voisine de 40 bars). Le taux de transformation de la trichloro-3,4,5 aniline est de 100 %. Le rendement en dichloro-3,5 aniline est de 99 %, celui de la métachloraniline de 1 %. Le taux d'hydrodéchloration du solvant est de 0,06 %.

### Exemple 4

On répète l'exemple 2 en remplaçant la trichloro-3,4,5 aniline par la tétrachloro-2,3,4,5 aniline et avec 0,4 g du même catalyseur Pd à 5 % au lieu de 0,2 g. Après 10 heures de réaction dans les conditions de l'exemple 2 le taux de transformation de la tétrachloro-2,3,4,5 aniline est de 100 %. Le rendement en dichloro-3,5 aniline est de 98,5 % et celui de la chloro-3 aniline est de 1,5 %. Le taux d'hydrodéchloration du solvant est de 0,06 %.

### Exemple 5

On opère comme il est dit dans l'exemple 1. Les charges sont :

| | |
|---|---|
| trichloro-3,4,5 aniline | $4,0 \times 10^{-3}$ moles |
| trichloro-1,2,4 benzène | 20 ml |
| catalyseur Pd à 5 %/charbon | 0,2 g |
| iode anhydre | $5,0 \times 10^{-5}$ atome gramme. |

On ne charge plus d'acide chlorhydrique et on pressurise l'autoclave sous 5 bars avec de l'hydrogène (mesurés dans les conditions ambiantes) soit environ 9 bars à 210 °C. Après 8 heures de réaction à 210 °C, on obtient, pour un taux de transformation de 94,6 %, un rendement de 96,8 % en dichloro-3,5 aniline par rapport à la trichloro-3,4,5 aniline transformée. Le taux d'hydrodéchloration du solvant est de 0,02 %.

### Exemple 6

On opère comme dans l'exemple 5 mais avec $5 \times 10^{-3}$ atome gramme d'iode. Après 7 heures de réaction à 210 °C, on obtient, pour un taux de transformation complet de la trichloro-3,4,5 aniline, un rendement de 98,8 % en dichloro-3,5 aniline. Il n'y a pas d'hydrodéchloration du solvant.

### Exemple 7

On répète l'exemple 1 en remplaçant la trichloro-3,4,5 aniline par la tétrachloro-2,3,5,6 aniline. On charge : $4,0 \times 10^{-3}$ mole de tétrachloro-2,3,5,6 aniline
0,25 g de catalyseur au Pd à 5 %/C
20 ml de trichloro-1,2,4 benzène
$6,2 \times 10^{-3}$ mole d'acide iodhydrique anhydre.

L'autoclave est pressurisé avec 6 bars d'hydrogène mesurés dans les conditions ambiantes et on laisse réagir 10 heures à 210 °C (la pression s'élève à 11 bars à 210 °C). Le traitement et l'analyse de l'essai sont effectués comme il est dit dans l'exemple 1. Pour un taux de transformation complet de la tétrachloro-2,3,5,6 aniline on obtient un rendement de 99,9 % en dichloro-3,5 aniline. Le taux d'hydrodéchloration du solvant est de 0,05 %.

### Exemple 8

On répète l'exemple 7 en chargeant $16,0 \times 10^{-3}$ de HI au lieu de $6,2 \times 10^{-3}$ mole. Après 2 heures de réaction dans ces mêmes conditions, le taux de transformation de la tétrachloro-2,3,5,6 aniline est de 98,5 %. On obtient la dichloro-3,5 aniline avec un rendement de 98,7 % par rapport à la tétrachloro-2,3,5,6 aniline transformée. Le taux d'hydrodéchloration du solvant est de 0,1 %.

Exemple 9

Dans un essai conduit comme l'exemple 1 on charge dans un autoclave de 250 ml en inox :

| | |
|---|---|
| trichloro-3,4,5 aniline | 0,0125 mole |
| trichloro-1,2,4 benzène | 20 ml |
| catalyseur au Pd/C à 5 % de Pd | 0,1 g |

$8 \times 10^{-3}$ mole d'acide bromhydrique anhydre et 5 bars d'hydrogène mesurés dans les conditions ambiantes (soit environ 9 bars à 210 °C).

Après 5 heures de réaction à 210 °C, on a obtenu, pour un taux de transformation complet de la trichloro-3,4,5 aniline, un rendement de 99 % en dichloro-3,5 aniline. Le taux d'hydrodéchloration du solvant est nul.

Exemple 10

On répète l'exemple 9 en remplaçant la trichloro-3,4,5 aniline par la dichloro-3,4 aniline. Après 7 heures de réaction dans les mêmes conditions on obtient la chloro-3 aniline avec un rendement de 100 % pour un taux de transformation de 100 % de la dichloro-3,4 aniline. Le taux d'hydrodéchloration du solvant est de 0,09 %.

Exemple 11

On reproduit l'exemple 9 en remplaçant la trichloro-3,4,5 aniline par la dichloro-2,3 aniline. Après 28 heures de réaction dans les mêmes conditions, on obtient la chloro-3 aniline avec un rendement de 100 % pour un taux de transformation de 100 % de la dichloro-2,3 aniline. Le taux d'hydrodéchloration du solvant est de 0,09 %.

Exemple 12

On reproduit l'exemple 9 en remplaçant la trichloro-3,4,5 aniline par la trichloro-2,4,5 aniline et en chargeant 0,5 g du même catalyseur au lieu de 0,1 g. Après 10 heures de réaction dans les mêmes conditions, on obtient la chloro-3 aniline avec un rendement de 100 % pour une transformation complète de la trichloro-2,4,5 aniline et des intermédiaires (dichloro-2,5-2,3-3,4 anilines). Le taux d'hydrodéchloration du solvant est de 0,09 %.

Exemple 13

Dans un autoclave de 225 $cm^3$ en acier inoxydable, on charge :

$1 \times 10^{-3}$ mole de trichloro-3,4,5 aniline,

40 ml de trichloro-1,2,4 benzène,

0,8 g d'un catalyseur, constitué de palladium déposé sur du charbon actif (surface spécifique 1 300 $m^2/g$, teneur pondérale en palladium 5 %).

On opère ensuite comme à l'exemple 1, si ce n'est qu'on introduit $2 \times 10^{-3}$ d'HCl et qu'on chauffe à 160 °C sous agitation pendant 10 heures.

Dans ces conditions, on observe que le taux de transformation de la trichloro-3,4,5 aniline est de 100 % et le rendement en dichloro-3,5 aniline est de 96,5 %. Il s'est également formé 2 % de chloro-3 aniline.

Exemple 14

Dans un autoclave de 225 $cm^3$ en acier inoxydable, on charge :

0,028 mole de dichloro-2,3 aniline,

0,012 mole de dichloro-3,4 aniline,

40 ml de dichloro-1,2 benzène,

0,45 ml d'une solution d'acide iodhydrique à 0,77 mole dans le dichloro-1,2 benzène ci-dessus (soit $0,35 \times 10^{-3}$ mole de HI),

0,32 g du catalyseur décrit à l'exemple 13.

On introduit 5 bars d'hydrogène à température ambiante puis on chauffe à 206 °C. A l'équilibre, la pression totale est de 12 bars. On laisse alors réagir pendant 15 h 30 mn.

Puis l'autoclave est refroidi, dégazé. Le mélange réactionnel est hydrolysé/neutralisé par une solution aqueuse de soude à 10 moles/l et la phase organique est analysée par chromatographie en phase vapeur.

Dans ces conditions, on observe que le taux de transformation de dichloro-3,4 aniline est de 100 %, celui de la dichloro-2,3 aniline de 62 %. Le rendement en chloro-3 aniline par rapport aux dichloroanilines transformées est de 99,9 %.

**Revendications**

1. Procédé de préparation d'anilines substituées en position méta par un atome d'halogène, par hydrogénation catalytique en phase liquide, organique, anhydre, à chaud et sous pression, en présence de métaux nobles du groupe VIII de la classification périodique, de dérivés benzéniques aminés et chlorés, de formule :

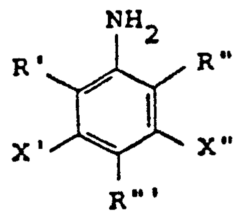

(I)

dans laquelle

X' et X", identiques ou différents entre eux représentent chacun un atome d'halogène, de préférence de chlore, ou un radical alcoyle de 1 à 4 atomes de carbone, ou alcoxyle de 1 à 4 atomes de carbone, au moins l'un des X' et X" étant obligatoirement un atome d'halogène, de préférence de chlore, l'un des X' et X" pouvant, de plus, être l'hydrogène,

R', R" et R"', identiques ou différents entre eux, représentent chacun un atome d'halogène, de préférence de chlore, ou un radical alcoyle de 1 à 4 atomes de carbone ou alcoxyle de 1 à 4 atomes de carbone, phényle ou benzyle éventuellement halogéné, l'un au moins de ces trois symboles représentant l'atome d'halogène, de

préférence de chlore, au plus deux des R', R", R"' pouvant de plus, être l'hydrogène,

caractérisé en ce que la réaction est effectuée en présence d'un hydracide notamment halogéné, ou d'un générateur de cet acide, dans un solvant chlorobenzénique.

2. Procédé selon la revendication 1), caractérisé en ce que l'hydracide halogéné est l'acide chlorhydrique.

3. Procédé selon la revendication 1, caractérisé en ce que l'hydracide halogéné est l'acide bromhydrique.

4. Procédé selon la revendication 1, caractérisé en ce que l'hydracide halogéné est l'acide iodhydrique.

5. Procédé selon l'une des revendications 1 à 4, caractérisé en ce que l'hydracide halogéné est dans un rapport molaire avec la polychloraniline de départ compris entre 1/100 et 10/1.

6. Procédé selon la revendication 5, caractérisé en ce que l'hydracide est dans un rapport molaire avec la polychloraniline de départ compris entre 1/10 et 5/1.

7. Procédé selon l'une des revendications 1 à 6, caractérisé en ce que le générateur d'hydracide est l'iode.

8. Procédé selon l'une des revendications 1 à 7, caractérisé en ce que le solvant est le chlorobenzène.

9. Procédé selon l'une des revendications 1 à 7, caractérisé en ce que le solvant est choisi dans le groupe comprenant le dichloro-1,2 et le dichloro-1,3 benzène.

10. Procédé selon l'une des revendications 1 à 7, caractérisé en ce que le solvant est trichloro-1,2,4 benzène.

11. Procédé selon l'une des revendications 1 à 10, caractérisé en ce que R', R", R"', X', X", identiques ou différents entre eux, représentent l'atome d'hydrogène ou l'atome de chlore.

12. Procédé de préparation de dimétachloroanilines, selon l'une des revendications 1 à 11, caractérisé en ce que X' et X" représentent l'atome de chlore.

13. Procédé de préparation de monométachloroanilines, selon l'une des revendications 1 à 12, caractérisé en ce que un seul des deux radicaux X' et X" est l'atome de chlore.

14. Procédé de préparation de dichloro-3,5 aniline selon l'une des revendications 1 à 9, caractérisé en ce que :

X' et X" sont l'atome de chlore,

R', R", R"' sont l'atome d'hydrogène ou l'atome de chlore.

15. Procédé selon l'une des revendications 1 à 14, caractérisé en ce que la pression totale est comprise entre 5 et 100 bars.

16. Procédé selon la revendication 15, caractérisé en ce que la pression totale est comprise entre 5 et 20 bars.

17. Procédé selon l'une des revendications 1 à 16, caractérisé en ce que la température est comprise entre 90 et 300 °C, de préférence entre 150 et 230 °C.

18. Procédé selon l'une des revendications 1 à 17, caractérisé en ce que le catalyseur est le palladium.

19. Procédé selon l'une des revendications 1 à 18, carctérisé en ce que la proportion pondérale de

métal noble par rapport au composé de formule (I) est comprise entre 0,01 et 10 %, de préférence entre 0,1 et 5 %.

**Claims**

1. A process for the preparation of anilines substituted in the meta-position by a halogen atom, by the catalytic hydrogenation, in an anhydrous organic liquid phase, under the action of heat and under pressure, in the presence of noble metals from group VIII of the periodic classification, of amine-substituted and chlorine-substituted benzene derivatives of the formula :

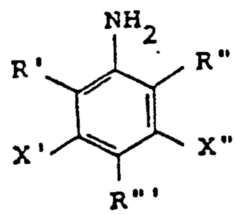

(I)

in which

X′ and X″, which are identical to or different from one another, each represent a halogen atom, preferably a chlorine atom, an alkyl radical having 1 to 4 carbon atoms or an alkoxy radical having 1 to 4 carbon atoms, at least one of X′ and X″ necessarily being a halogen atom, preferably a chlorine atom, and it also being possible for one of X′ and X″ to be hydrogen, and

R′, R″ and R‴, which are identical to or different from one another, each represent a halogen atom, preferably a chlorine atom, an alkyl radical having 1 to 4 carbon atoms, an alkoxy radical having 1 to 4 carbon atoms, an optionally halogenated phenyl or benzyl, or a phenoxy radical,
at least one of these three symbols representing the halogen atom, preferably the chlorine atom, and it also being possible for at most two of R′, R″ and R‴ to be hydrogen,
wherein the reaction is carried out in the presence of a hydracid, in particular a halogen-containing hydracid, or of a generator of this acid, in a chlorobenzene solvent.

2. A process according to claim 1, wherein the halogen-containing hydracid is hydrochloric acid.

3. A process according to claim 1, wherein the halogen-containing hydracid is hydrobromic acid.

4. A process according to claim 1, wherein the halogen-containing hydracid is hydriodic acid.

5. A process according to one of claims 1 to 4, wherein the halogen-containing hydracid is in a molar ratio to the starting polychloroaniline of between 1/100 and 10/1.

6. A process according to claim 5, wherein the hydracid is in a molar ratio to the starting polychloroaniline of between 1/10 and 5/1.

7. A process according to one of claims 1 to 6, wherein the hydracid generator is iodine.

8. A process according to one of claims 1 to 7, wherein the solvent is chlorobenzene.

9. A process according to one of claims 1 to 7, wherein the solvent is chosen from the group comprising 1,2-dichlorobenzene and 1,3-dichlorobenzene.

10. A process according to one of claims 1 to 7, wherein the solvent is 1,2,4-trichlorobenzene.

11. A process according to one of claims 1 to 10, in which R′, R″, R‴, X′ and X″, which are identical to or different from one another, represent the hydrogen atom or the chlorine atom.

12. A process for the preparation of meta-dichloroanilines according to one of claims 1 to 11, in which X′ and X″ represent the chlorine atom.

13. A process for the preparation of meta-monochloroanilines according to one of claims 1 to 12, in which only one of the two radicals X′ and X″ is the chlorine atom.

14. A process for the preparation of 3,5-dichloroaniline according to one of claims 1 to 9, in which
X′ and X″ are the chlorine atom and
R′, R″ and R‴ are the hydrogen atom or the chlorine atom.

15. A process according to one of claims 1 to 14, wherein the total pressure is between 5 and 100 bars.

16. A process according to claim 15, wherein the total pressure is between 5 and 20 bars.

17. A process according to one of claims 1 to 16, wherein the temperature is between 90 and 300 °C, preferably between 150 and 230 °C.

18. A process according to one of claims 1 to 17, wherein the catalyst is palladium.

19. A process according to one of claims 1 to 18, wherein the proportion by weight of noble metal, relative to the compound of the formule (I), is between 0.01 and 10 %, preferably between 0.1 and 5 %.

**Patentansprüche**

1. Verfahren zur Herstellung m-halogenierter Aniline durch katalytische Hydrierung halogenierter Anilinderivate der Formel I

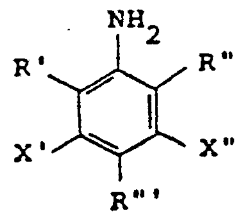

(I)

in der bedeuten

X' und X" zugleich oder unabhängig Halogen, vorzugsweise Chlor, $C_1$-bis $C_4$-Alkyl oder $C_1$- bis $C_4$-Alkoxy, wobei mindestens einer der Substituenten X' und X" zwingend Halogen und vorzugsweise Chlor ist und einer der Substituenten X' und X" ferner Wasserstoff sein kann, und

R', R" und R"' zugleich oder unabhängig Halogen, vorzugsweise Chlor, $C_1$- bis $C_4$-Alkyl, $C_1$- bis $C_4$-Alkoxy oder Phenyl, Benzyl,

die gegebenenfalls halogeniert sind, oder Phenoxy

wobei mindestens einer dieser drei Substituenten Halogen und vorzugsweise Chlor ist und höchstens zwei der Substituenten R', R" und R"' ferner Wasserstoff bedeuten können,

in flüssiger, organischer wasserfreier Phase in der Wärme und unter Druck in Gegenwart von Edelmetallen der Gruppe VIII des Periodensystems,

dadurch gekennzeichnet, daß die Reaktion in Gegenwart einer Protonsäure nämlich einer halogenhaltigen Protonsäure oder einer eine Protonsäure freisetzenden Verbindung in einem chlorierten Benzol als Lösungsmittel durchgeführt wird.

2. Verfahren nach Anspruch 1, dadurch gekennzeichnet, daß die Reaktion in Gegenwart von Chlorwasserstoffsäure als halogenhaltiger Protonsäure durchgeführt wird.

3. Verfahren nach Anspruch 1, dadurch gekennzeichnet, daß die Reaktion in Gegenwart von Bromwasserstoffsäure als halogenhaltiger Protonsäure durchgeführt wird.

4. Verfahren nach Anspruch 1, dadurch gekennzeichnet, daß die Reaktion in Gegenwart von Jodwasserstoffsäure als halogenhaltiger Protonsäure durchgeführt wird.

5. Verfahren nach einem der Ansprüche 1 bis 4, dadurch gekennzeichnet, daß die halogenhaltige Protonsäure in einem Molverhältnis zum Polychloroanilin der Formel (I) von 1 : 100 bis 10 : 1 eingesetzt wird.

6. Verfahren nach Anspruch 5, dadurch gekennzeichnet, daß die Protonsäure in einem Molverhältnis zum Polychloroanilin der Formel I von 1 : 10 bis 5 : 1 eingesetzt wird.

7. Verfahren nach einem der Ansprüche 1 bis 6, dadurch gekennzeichnet, daß als eine Protonsäure freisetzende Verbindung Jod verwendet wird.

8. Verfahren nach einem der Ansprüche 1 bis 7, dadurch gekennzeichnet, daß als Lösungsmittel Chlorbenzol verwendet wird.

9. Verfahren nach einem der Ansprüche 1 bis 7, dadurch gekennzeichnet, daß als Lösungsmittel 1.2-Dichlorbenzol oder 1.3-Dichlorbenzol verwendet werden.

10. Verfahren nach einem der Ansprüche 1 bis 7, dadurch gekennzeichnet, daß Lösungsmittel 1.2.4-Trichlorbenzol verwendet wird.

11. Verfahren nach einem der Ansprüche 1 bis 10, dadurch gekennzeichnet, daß R', R", R"', X' und X" zugleich oder unabhängig Wasserstoff oder Chlor bedeuten.

12. Verfahren nach einem der Ansprüche 1 bis 11 zur Herstellung von Di-m-chloranilinen, dadurch gekennzeichnet, daß X' und X" Chlor bedeuten.

13. Verfahren nach einem der Ansprüche 1 bis 11 zur Herstellung von Mono-m-chloranilinen, dadurch gekennzeichnet, daß nur einer der Substituenten X' und X" Chlor bedeutet.

14. Verfahren nach einem der Ansprüche 1 bis 9, dadurch gekennzeichnet, daß ein halogeniertes Anilinderivat der Formel I verwendet wird, in der X' und X" Chlor und R', R" und R"' Wasserstoff oder Chlor bedeuten.

15. Verfahren nach einem der Ansprüche 1 bis 14, dadurch gekennzeichnet, daß die Reaktion bei einem Gesamtdruck von 5 bis 100 bar durchgeführt wird.

16. Verfahren nach Anspruch 15, dadurch gekennzeichnet, daß die Reaktion bei einem Gesamtdruck von 5 bis 20 bar durchgeführt wird.

17. Verfahren nach einem der Ansprüche 1 bis 16, dadurch gekennzeichnet, daß die Reaktion bei einer Temperatur von 90 bis 300 °C, vorzugsweise von 150 bis 230 °C, durchgeführt wird.

18. Verfahren nach einem der Ansprüche 1 bis 17, dadurch gekennzeichnet, daß als Katalysator Palladium verwendet wird.

19. Verfahren nach einem der Ansprüche 1 bis 18, dadurch gekennzeichnet, daß das Edelmetall in einer Menge von 0,01 bis 10 Gew.-%, vorzugsweise von 0,1 bis 5 Gew.-%, bezogen auf das Derivat der Formel I, eingesetzt wird.